# EUROPEAN PATENT APPLICATION

(11) **EP 4 566 602 A1**
(43) Date of publication of application: **11.06.2025**
(21) Application number: 23849257.3
(22) Date of filing: 27.07.2023
(51) Int. Cl.: A61K 31/40, A61K 9/08, A61P 1/00, A61P 1/04, A61P 1/14, A61P 31/04

(54) **PHARMACEUTICAL COMPOSITION CONTAINING PYRROLE GASTRIC ACID SECRETION INHIBITOR AND PREPARATION METHOD THEREFOR**

(30) Priority: 04.08.2022 CN 202210930967
(71) Applicant: Jiangsu Carephar Pharmaceutical Co., Ltd., Nanjing, Jiangsu 210016 (CN)
(72) Inventor: SU, Mei, Nanjing, Jiangsu 210016 (CN); HAN, Cheng, Nanjing, Jiangsu 210016 (CN); SHEN, Juan, Nanjing, Jiangsu 210016 (CN)
(74) Representative: karo IP
(86) International application number: PCT/CN2023/109519
(87) International publication number: WO 2024/027549

(57) **Abstract**

A pharmaceutical composition containing a pyrrole gastric acid secretion inhibitor and a preparation method therefor are provided. The pharmaceutical composition includes a pharmaceutically acceptable salt of a compound represented by formula I, and a stabilizer. Also provided is a method for preparing the pharmaceutical composition. The method is simple, cost-effective, and suitable for industrial mass production. At present, no injectable composition of the pharmaceutical compound is available clinically.

## Description

### TECHNICAL FIELD

The present disclosure relates to an injectable composition and a preparation method therefor, and in particular, to an injectable composition containing a pyrrole gastric acid secretion inhibitor and a preparation method therefor.

### BACKGROUND

Proton pump inhibitors are widely used clinically to treat peptic ulcer, reflux esophagitis, and Zollinger-Ellison syndrome by inhibiting gastric acid secretion. Potassium-competitive acid blockers (P-CABs) are a new class of acid inhibiting drugs, which competitively and reversibly bind to potassium-binding sites in proton pumps on gastric parietal cells. In an acidic environment, P-CABs are immediately ionized and bind ionically to the H⁺-K⁺-ATPase. P-CABs do not need to concentrate in the microcapsules and microtubules of the gastric parietal cells, do not require activation by acid, and can quickly increase the pH in the stomach. The enzyme activity is restored after dissociation. P-CABs have a strong inhibitory effect on the mucosal damage of the upper gastrointestinal tract and are used for treating gastric ulcer, duodenal ulcer, reflux esophagitis, etc.

At present, there are no proton pump inhibitors in the form of an injection solution clinically. Existing forms of proton pump inhibitors mainly include oral solid preparations and lyophilized powder for injection. Proton pump inhibitors in the form of oral solid preparations, such as tablets, have poor compliance for patients with gastrointestinal bleeding or acute peptic ulcer who require quick onset of therapeutic action, and patients who do not tolerate oral administration, and cannot meet the clinical requirement for rapid onset of therapeutic action. The preparations in the form of a lyophilized powder for injection can solve the above problems, but requires the use of a large number of excipients and higher costs of packaging materials. Due to the stability of raw materials, terminal sterilization with the highest level of sterility assurance cannot be applied to the preparations in the form of a lyophilized powder for injection, leading to high production costs of the preparation process. Moreover, the preparations in the form of a lyophilized powder for injection have more stringent requirements on storage conditions, and need to be stored at a low temperature.

The chemical name of the compound represented by formula I is 1-[5-(2-fluoro-phenyl)-1-{[3-(3-methoxypropoxy) phenyl]sulfonyl}-1H-pyrrol-3-yl]-N-methylmethylamine, which is a class of P-CABs. It can be used for the prevention and treatment of gastric acid-related diseases including, but not limited to, gastrointestinal diseases such as peptic ulcer, Zollinger-Ellison syndrome, gastritis, gastric ulcer, duodenal ulcer, ulcer caused by non-steroidal anti-inflammatory drugs, Helicobacter pylori infection, gastroesophageal reflux disease, reflux esophagitis, and the like.

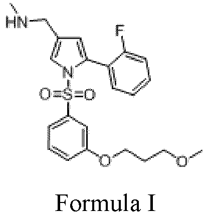

Chinese Patent CN 108969520A discloses a composition of a crystal A compound of N-methylformamide hydrochloride, which is a hydrochloride salt formed by reaction of the compound of formula I with hydrochloric acid and is represented by formula II, and specifically discloses an oral composition containing the compound represented by formula II. The oral composition has poor compliance for patients with gastrointestinal bleeding or acute peptic ulcer who require quick onset of therapeutic action, and patients who do not tolerate oral administration, and cannot meet the clinical requirement for rapid onset of therapeutic action. Compared with tablets, injections have the advantages of high bioavailability and rapid onset of therapeutic action.

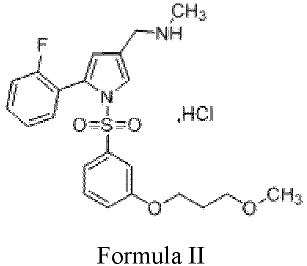

The hydrochloride salt represented by formula II is likely to degrade to form impurities during preparation and storage. Impurities known to be formed by degradation mainly include impurity 1 and impurity 2, the structures of which are respectively as follows:

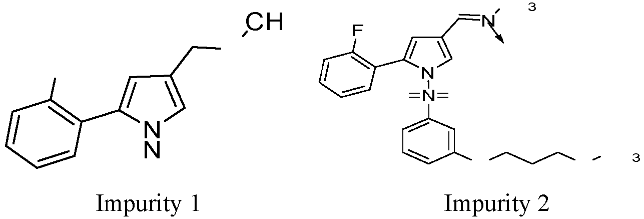

The hydrochloride salt represented by formula II is a new class of P-CABs, and at present, there is no proton pump inhibitor in the form of an injection solution clinically. To prepare the hydrochloride salt represented by formula II into an injection solution, it is necessary to solve the solubility problem, and reduce the types and amounts of excipients used as much as possible, to reduce the adverse reactions caused by excipients in the preparation to human body and improve safety. In addition, it is necessary to control the content of related impurities during production and storage to improve the stability of the preparation containing the composition.

### SUMMARY

A first aspect of the present disclosure provides an injectable composition, including a pharmaceutically acceptable salt of a compound represented by formula I, and a stabilizer, where the stabilizer is an edetate.

In some embodiments, the edetate is one or more of disodium edetate, calcium disodium edetate, tetrasodium edetate, or dicalcium edetate. In some specific embodiments, the stabilizer is disodium edetate. In some specific embodiments, the stabilizer is calcium disodium edetate.

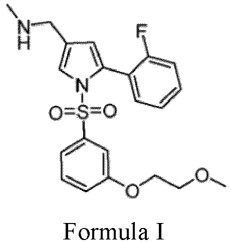

In some embodiments, the pharmaceutically acceptable salt of the compound represented by formula I is fumarate, oxalate, succinate, lactate, mesylate, L-malate, citrate, nitrate, hydrobromide, hydrochloride, phosphate, or sulfate. In some specific embodiments, the salt of the compound represented by formula I may be a hydrochloride represented by formula II below.

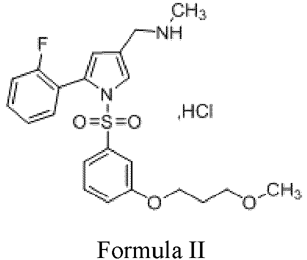

In some embodiments, the injectable composition of the present disclosure includes the pharmaceutically acceptable salt of the compound represented by formula I and the stabilizer, and the weight ratio of the pharmaceutically acceptable salt of the compound represented by formula I to the stabilizer is 1:1 to 20:1. In some embodiments, the weight ratio of the pharmaceutically acceptable salt of the compound of formula I to the stabilizer may be 1:1, 2:1, 3:1, 4:1, 5:1, 6:1, 7:1, 8:1, 9:1, 10:1, 15:1, or 20:1, or any range defined thereby, for example, preferably 5:1 to 20:1, 5:1 to 15:1, or 5:1 to 10:1, etc. The weight ratio herein is based on the hydrochloride represented by formula II, and the corresponding weight ratio range of the compound represented by formula I or other salts in the compound other than the hydrochloride can be calculated by conversion.

In some embodiments, the pH of the injectable composition of the present disclosure is a pharmaceutically acceptable pH. In some embodiments, the pH of the composition is 3.0 to 4.5. In some embodiments, the pH of the composition is 3.0 to 4.0. In some embodiments, the pH of the composition is 3.5. In some embodiments, the pH of the composition is 4.0. In some embodiments, the pH of the composition is 4.5. The composition of the present disclosure may be adjusted according to conventional methods in the art.

In some embodiments, a pH regulator or the like may be added to adjust the pH of the composition of the present disclosure. During the preparation of the injectable composition, a conventional pH regulator for adjusting the pH of the composition may be added. For example, the pH regulator is one or more of hydrochloric acid, sodium hydroxide, citric acid, phosphoric acid, fumaric acid, tartaric acid, or sodium bicarbonate. In some specific embodiments, the pH regulator is hydrochloric acid.

The amount of the active ingredient in the injectable composition of the present disclosure varies with usage conditions of the target patient, the degree of targeted therapy, and the like. In some embodiments, the concentration of the hydrochloride represented by formula II in the injectable composition is 1 mg/mL to 20 mg/mL. In some embodiments, 1 mg to 10 mg of the hydrochloride represented by formula II is contained per 1 mL of the composition. In some embodiments, 1 mg to 5 mg of the hydrochloride represented by formula II is contained per 1 mL of the composition. In some embodiments, 1 mg, 2 mg, 2.5 mg, 3 mg, 3.5 mg, 4 mg, 4.5 mg, or 5 mg of the hydrochloride represented by formula II is contained per 1 mL of the composition. For example, the injectable composition contains 2.5 mg (or any specific value in the range defined in the present disclosure) of the compound represented by formula II and 0.125 mg to 0.5 mg, preferably 0.25 mg to 0.5 mg, of the stabilizer per 1 mL of the injectable composition.

The injectable composition of the pharmaceutically acceptable salt of the compound of formula I disclosed in the present disclosure may not include additives other than the stabilizer and the pH regulator. For example, the injectable composition includes the pharmaceutically acceptable salt of the compound represented by formula I, the stabilizer, a pH regulator, and water for injection, where the active ingredient concentration of the pharmaceutically acceptable salt of the compound of formula I is 1 mg/mL to 20 mg/mL; the weight ratio of the pharmaceutically acceptable salt of the compound of formula I to the edetate is 1:1 to 20:1, preferably 5:1 to 10:1; and the injectable composition has a pH of 3.0 to 4.5. The pH regulator is used in an amount sufficient to maintain the pH value of the composition.

The injectable composition of the pharmaceutically acceptable salt of the compound of formula I disclosed herein may also optionally include, but is not limited to, an excipient conventionally used in the art, such as an osmotic pressure regulator, a buffer solution, and the like. The osmotic pressure regulator may be one of sodium chloride or glucose. The ratio of the weight of the osmotic pressure regulator to the total volume of the injection solution is 0.6% to 1.0% (W/V, g/100 mL). In some embodiments, the osmotic pressure regulator is sodium chloride. In some embodiments, the ratio of the weight of sodium chloride to the total volume of the injection solution is 0.6%, 0.7%, 0.8%%, 0.9%, or 1.0%.

The injectable composition of the present disclosure may be prepared as a solution-type injection. In some embodiments, the injection is a large-volume injection. In some embodiments, the injection is a small-volume injection.

The present disclosure also provides a composition including the injection solution and an infusion solution. The infusion solution may be an electrolyte solution (e.g., saline, Ringer's solution, etc.), a nutrient solution (e.g., a carbohydrate solution (such as a glucose solution, e.g., 5% (w/v) glucose solution)), and the like. The composition of the present disclosure has simple ingredients and a safe process. By preparing the pharmaceutically acceptable salt of the compound of formula I into the injection composition with the addition of a certain amount of the stabilizer to control the pH of the composition, good physical and chemical stability of the composition can be guaranteed, the impurity content is easily controlled and does not significantly increase, the quality and solubility requirements of the injection are met, no additional excipient such as a co-solvent or a solubilizing agent needs to be added, and the safety of the composition is improved.

The smaller the number of types and dosage of the excipients used in the composition of the present disclosure, the higher the safety to human body after injection. The injection composition of the present disclosure can have good stability with a small amount of the stabilizer, so the content of impurities 1 and 2 is effectively reduced.

The injection composition has higher safety and good storage stability, providing a new medication choice to patients who do not tolerate oral administration, to meet the clinical requirements of patients with gastrointestinal bleeding or acute peptic ulcer who require quick onset of therapeutic action.

A second aspect of the present disclosure provides a method for preparing the composition described above. The method is simple and easy, adopts terminal sterilization, has good stability and high safety, and is suitable for industrial production.

To achieve the second objective of the present disclosure, the following technical solution is adopted in the present disclosure. A method for preparing the composition of the pharmaceutically acceptable salt of the compound represented by formula I is provided, which includes the following steps:
dissolving the pharmaceutically acceptable salt of the compound of formula I and the stabilizer in a volume of water for injection, followed by filling, sealing, and moist heat sterilization to obtain the composition.

Further, the pharmaceutically acceptable salt of the compound of formula I and the stabilizer are dissolved in the volume of water for injection; the pH of the solution is adjusted to 3.0 to 4.5 with a pH regulator, followed by filling and sealing; and the solution is sterilized by moist heat sterilization for 15 minutes to 30 minutes.

The stabilizer is an edetate. The edetate is, for example, one or more of disodium edetate, calcium disodium edetate, tetrasodium edetate, or dicalcium edetate. In some embodiments, the stabilizer is disodium edetate. In some embodiments, the stabilizer is calcium disodium edetate.

In some embodiments, the pharmaceutically acceptable salt of the compound of formula I is hydrochloride, represented by formula II.

In some embodiments, the hydrochloride represented by formula II and calcium disodium edetate or disodium edetate are dissolved in the volume of water for injection; the pH of the solution is adjusted to 3.0 to 4.5 with a pH regulator, and water for injection is added to make up to volume; and the solution is sterilized by moist heat sterilization at 121°C for 15 minutes to 30 minutes.

In some embodiments, the hydrochloride represented by formula II and calcium disodium edetate or disodium edetate are added to 80% to 90% of an amount of water for injection for a production batch, and stirred until dissolved; the pH of the solution is adjusted to 3.0 to 4.5 with 1 mol/L hydrochloric acid, and water for injection is added to the full amount; and the solution is filtered and flushed with nitrogen, followed by filling, sealing, and moist heat sterilization at 121°C for 15 minutes.

The process of preparing an injection solution in the present disclosure is simple, involves the use of only a small number of types and a small amount of excipients, adopts terminal sterilization, and does not require the use of activated carbon, thereby reducing production costs and achieving high clinical safety.

A third aspect of the present disclosure provides a specific use of the composition. The pharmaceutically acceptable salt of the compound of formula I or the compound of formula II is used as a pharmaceutically active ingredient to form a composition. The composition may be used in preparation of a medicament for treatment or prevention of peptic ulcer (e.g., gastric ulcer, duodenal ulcer, anastomotic ulcer, etc.), Zollinger-Ellison syndrome, gastritis, erosive esophagitis, reflux esophagitis, symptomatic gastroesophageal reflux disease (e.g., non-erosive reflux disease or gastroesophageal reflux disease without esophagitis), Barrett's esophagitis, functional dyspepsia, Helicobacter pylori infection, gastric cancer, gastric mucosa-assisted lymphoid tissue (MALT) lymphoma, ulcer caused by non-steroidal anti-inflammatory drugs, or hyperchlorhydria or ulcer caused by postoperative stress; or in preparation of a medicament for inhibiting peptic ulcer, acute stress ulcer, hemorrhagic gastritis, or upper gastrointestinal bleeding caused by invasive stress.

The injectable composition of the present disclosure or the injection prepared from the injectable composition have a rapid hemostatic effect on upper gastrointestinal bleeding, and are useful for patients with upper gastrointestinal bleeding who do not tolerate oral administration.

### DETAILED DESCRIPTION

The following examples are provided for a better understanding of the present disclosure; however, the present disclosure is not limited thereto. The methods given in examples below are all conventional methods, unless it is otherwise stated. Test materials used in the following examples are commercially available, unless otherwise specified.

### Examples 1 to 6

The compound represented by formula II and the stabilizer were weighed and dissolved in 90% of a prescribed amount of water for injection. The pH of the solution was adjusted with hydrochloric acid, and then water for injection was added to the full amount. The solution was filled up to 2 mL, and was flushed with nitrogen before and after filling and sealing. The solution was sterilized at 121°C for 15 minutes to obtain the composition. The characteristics of samples from different examples and the changes of impurities were investigated. The changes of impurities were detected by high-performance liquid chromatography (HPLC). The analytical method used to detect the impurity content was as follows:
Chromatographic column: with octadecylsilane bonded silica gel as the filler; Detector: UV detector; Detection wavelength: 230 nm; Mobile phase: 0.01 mol/L dipotassium hydrogen phosphate buffer-acetonitrile.

The impurity test results are expressed by calculating the peak areas (%) of the impurities by principal component self-control. See Table 1 for specific examples and investigation results.

**Table 1**

| Ingredient | | | Example 1 | Example 2 | Example 3 | Example 4 | Example 5 | Example 6 |
|---|---|---|---|---|---|---|---|---|
| Compound represented by formula II (g) | | | 2.0 | 2.0 | 2.0 | 2.0 | 2.0 | 2.0 |
| Sodium metabisulfite (g) | | | 0.4 | / | / | / | / | / |
| Citric acid (g) | | | / | 0.4 | / | / | / | / |
| Disodium edetate (g) | | | / | / | 0.4 | / | / | / |
| Calcium disodium edetate (g) | | | / | / | / | 0.4 | / | / |
| vc (g) | | | / | / | / | / | 0.4 | / |
| Hydrochloric acid | | | Appropriate amount | Appropriate amount | Appropriate amount | Appropriate amount | Appropriate amount | Appropriate amount |
| pH | | | 3.5 | 3.5 | 3.5 | 3.5 | 3.5 | 3.5 |
| Add water for injection to make the total volume (mL) | | | 200 | 200 | 200 | 200 | 200 | 200 |
| Day 0 | Property | | Clear, colorless liquid | Clear, colorless liquid | Clear, colorless liquid | Clear, colorless liquid | Clear, colorless liquid | Clear, colorless liquid |
| | Impurities (%) | Impurity 1 | 0.04 | 0.05 | Not detected | 0.03 | 0.05 | 0.07 |
| | | Impurity 2 | 0.02 | 0.03 | Not detected | Not detected | 0.02 | 0.04 |
| | | Maximum single impurity | 0.08 | 0.09 | 0.07 | 0.07 | 0.07 | 0.09 |
| | | Total impurities | 0.25 | 0.23 | 0.18 | 0.19 | 0.26 | 0.29 |
| 60°C-Day 10 | Property | | Clear, colorless liquid | Clear, colorless liquid | Clear, colorless liquid | Clear, colorless liquid | Clear, colorless liquid | Clear, colorless liquid |
| | Impurities (%) | Impurity 1 | 0.06 | 0.07 | 0.04 | 0.05 | 0.07 | 0.08 |
| | | Impurity 2 | 0.04 | 0.05 | 0.02 | Not detected | 0.04 | 0.05 |
| | | Maximum single impurity | 0.09 | 0.09 | 0.07 | 0.07 | 0.08 | 0.11 |
| | | Total impurities | 0.29 | 0.28 | 0.20 | 0.21 | 0.32 | 0.37 |
| Illumination-Day 10 | Property | | Clear, colorless liquid | Clear, colorless liquid | Clear, colorless liquid | Clear, colorless liquid | Clear, light yellow liquid | Light yellow suspension |
| | Impurities (%) | Impurity 1 | 0.32 | 0.29 | Not detected | 0.03 | 0.36 | 0.56 |
| | | Impurity 2 | 0.18 | 0.15 | Not detected | Not detected | 0.22 | 0.41 |
| | | Maximum single impurity | 0.29 | 0.22 | 0.08 | 0.08 | 0.33 | 0.37 |
| | | Total impurities | 1.49 | 1.37 | 0.61 | 0.66 | 2.11 | 4.17 |

As shown in Table 1, at Day 0 and Day 10 of storage of the compositions including different stabilizers at a high temperature of 60°C, the properties of the samples did not change, and the changes of impurities were small. After the compositions were illuminated at 5000 Lx for 10 days, the properties of the compositions including no stabilizer changed from a clear, colorless liquid to a light yellow suspension, and the related substances increased significantly. The addition of the stabilizers into the compositions improved the stability of the compositions to different extents. With the use of the disodium edetate and the calcium disodium edetate as the stabilizer, the injectable compositions had significantly increased stability and good properties, and especially the growth of impurities 1 and 2 was significantly reduced.

### Examples 7 to 16

The compound represented by formula II and disodium edetate or calcium disodium edetate were weighed and dissolved in 90% of a prescribed amount of water for injection. The pH of the solution was adjusted with hydrochloric acid, and then water for injection was added to the full amount. The solution was filled in a 2 mL ampoule, and was flushed with nitrogen before and after filling and sealing. The sample was sterilized at 121°C for 15 minutes to obtain the composition. The compositions were placed under conditions including a high temperature of 60°C and illumination at 5000 Lx for stability investigation, to determine the properties of the injection solutions and the changes of impurities. The results are as shown in Table 2 to Table 3.

**Table 2**

| Ingredient | | | Example 7 | Example 8 | Example 9 | Example 10 | Example 11 |
|---|---|---|---|---|---|---|---|
| Compound represented by formula II (g) | | | 1.0 | 1.0 | 1.0 | 1.0 | 1.0 |
| Disodium edetate (g) | | | 0.02 | 0.05 | 0.1 | 0.5 | 1.0 |
| Hydrochloric acid | | | Appropriate amount | Appropriate amount | Appropriate amount | Appropriate amount | Appropriate amount |
| pH | | | 4.0 | 4.0 | 4.0 | 4.0 | 4.0 |
| Add water for injection to make the total volume (mL) | | | 200 | 200 | 200 | 200 | 200 |
| Day 0 | Property | | Clear, colorless liquid | Clear, colorless liquid | Clear, colorless liquid | Clear, colorless liquid | Clear, colorless liquid |
| | Impurities (%) | Impurity 1 | 0.21 | 0.06 | Not detected | 0.03 | Not detected |
| | | Impurity 2 | 0.15 | 0.02 | Not detected | Not detected | Not detected |
| | | Maximum single impurity | 0.26 | 0.08 | 0.06 | 0.07 | 0.08 |
| | | Total impurities | 1.06 | 0.31 | 0.22 | 0.19 | 0.21 |
| 60°C-Day 10 | Property | | Clear, colorless liquid | Clear, colorless liquid | Clear, colorless liquid | Clear, colorless liquid | Clear, colorless liquid |
| | Impurities (%) | Impurity 1 | 0.35 | 0.07 | 0.04 | 0.05 | 0.05 |
| | | Impurity 2 | 0.22 | 0.03 | 0.02 | Not detected | 0.02 |
| | | Maximum single impurity | 0.31 | 0.06 | 0.06 | 0.06 | 0.07 |
| | | Total impurities | 1.76 | 0.39 | 0.27 | 0.22 | 0.25 |
| Illumination-Day 10 | Property | | Clear, light yellow liquid | Clear, colorless liquid | Clear, colorless liquid | Clear, colorless liquid | Clear, colorless liquid |
| | Impurities (%) | Impurity 1 | 0.52 | 0.09 | 0.07 | 0.07 | 0.07 |
| | | Impurity 2 | 0.37 | 0.07 | 0.04 | 0.05 | 0.04 |
| | | Maximum single impurity | 0.48 | 0.10 | 0.08 | 0.08 | 0.08 |
| | | Total impurities | 2.46 | 0.71 | 0.65 | 0.63 | 0.62 |

**Table 3**

| Ingredient | | | Example 12 | Example 13 | Example 14 | Example 15 | Example 16 |
|---|---|---|---|---|---|---|---|
| Compound represented by formula II (g) | | | 1.0 | 1.0 | 1.0 | 1.0 | 1.0 |
| Calcium disodium edetate (g) | | | 0.02 | 0.05 | 0.1 | 0.5 | 1.0 |
| Hydrochloric acid | | | Appropriate amount | Appropriate amount | Appropriate amount | Appropriate amount | Appropriate amount |
| pH | | | 4.0 | 4.0 | 4.0 | 4.0 | 4.0 |
| Add water for injection to make the total volume (mL) | | | 200 | 200 | 200 | 200 | 200 |
| Day 0 | Property | | Clear, colorless liquid | Clear, colorless liquid | Clear, colorless liquid | Clear, colorless liquid | Clear, colorless liquid |
| | Impurities (%) | Impurity 1 | 0.24 | 0.07 | 0.04 | 0.02 | 0.04 |
| | | Impurity 2 | 0.17 | 0.04 | Not detected | Not detected | Not detected |
| | | Maximum single impurity | 0.28 | 0.06 | 0.06 | 0.07 | 0.07 |
| | | Total impurities | 1.29 | 0.34 | 0.24 | 0.20 | 0.23 |
| 60°C-Day 10 | Property | | Clear, colorless liquid | Clear, colorless liquid | Clear, colorless liquid | Clear, colorless liquid | Clear, colorless liquid |
| | Impurities (%) | Impurity 1 | 0.39 | 0.09 | 0.04 | 0.04 | 0.05 |
| | | Impurity 2 | 0.26 | 0.05 | Not detected | Not detected | Not detected |
| | | Maximum single impurity | 0.37 | 0.08 | 0.07 | 0.07 | 0.07 |
| | | Total impurities | 1.84 | 0.44 | 0.29 | 0.25 | 0.26 |
| Illumination-Day 10 | Property | | Clear, light yellow liquid | Clear, colorless liquid | Clear, colorless liquid | Clear, colorless liquid | Clear, colorless liquid |
| | Impurities (%) | Impurity 1 | 0.49 | 0.1 | 0.08 | 0.08 | 0.08 |
| | | Impurity 2 | 0.36 | 0.07 | 0.05 | 0.05 | 0.04 |
| | | Maximum single impurity | 0.46 | 0.12 | 0.07 | 0.07 | 0.06 |
| | | Total impurities | 2.54 | 0.79 | 0.63 | 0.61 | 0.65 |

As shown in Table 2 and Table 3, when the mass ratio of the stabilizer to the compound represented by formula II was 0.02, the stability of the composition was poor, impurities in the sample were significantly increased under high temperature and illumination conditions, and the color of the composition changed from colorless to light yellow under illumination. When the mass ratio of the stabilizer to the compound represented by formula II was in the range of 0.05 to 1.0 as described in the present disclosure, the injectable composition had good stability under high temperature and illumination conditions, and the property of the injectable composition was not significantly changed.

### Examples 17 to 21

The compound represented by formula II and disodium edetate or calcium disodium edetate were weighed and dissolved in 90% of a prescribed amount of water for injection. The pH of the solution was adjusted with hydrochloric acid, and then water for injection was added to the full amount. The solution was filled in a 2 mL ampoule, and was flushed with nitrogen before and after filling and sealing. The sample was sterilized at 121°C for 15 minutes to obtain the composition. The compositions were placed under conditions including a high temperature of 60°C and illumination at 5000 Lx for stability investigation, to determine the properties of the injection solutions and the changes of impurities. The results are as shown in Table 4.

**Table 4**

| Ingredient | | | Example 17 | Example 18 | Example 19 | Example 20 | Example 21 |
|---|---|---|---|---|---|---|---|
| Compound represented by formula II (g) | | | 3.0 | 3.0 | 3.0 | 3.0 | 3.0 |
| Disodium edetate (g) | | | 0.6 | / | 1.5 | / | 1.2 |
| Calcium disodium edetate (g) | | | / | 0.9 | / | 1.8 | / |
| Hydrochloric acid | | | / | Appropriate amount | / | Appropriate amount | / |
| Phosphoric acid | | | Appropriate amount | / | Appropriate amount | / | Appropriate amount |
| pH | | | 2.0 | 3.0 | 4.0 | 4.5 | 5.0 |
| Add water for injection to make the total volume (mL) | | | 200 | 200 | 200 | 200 | 200 |
| Day 0 | Property | | Precipitated crystals observed | Clear, colorless liquid | Clear, colorless liquid | Clear, colorless liquid | Clear, colorless liquid |
| | Impurities (%) | Impurity 1 | / | 0.03 | 0.04 | 0.06 | 0.18 |
| | | Impurity 2 | / | Not detected | Not detected | 0.04 | 0.12 |
| | | Maximum single impurity | / | 0.06 | 0.06 | 0.08 | 0.14 |
| | | Total impurities | / | 0.19 | 0.22 | 0.36 | 0.72 |
| 60°C-Day 10 | Property | | / | Clear, colorless liquid | Clear, colorless liquid | Clear, colorless liquid | Clear, colorless liquid |
| | Impurities (%) | Impurity 1 | / | 0.04 | 0.04 | 0.08 | 0.39 |
| | | Impurity 2 | / | Not detected | Not detected | 0.05 | 0.26 |
| | | Maximum single impurity | / | 0.07 | 0.07 | 0.09 | 0.31 |
| | | Total impurities | / | 0.24 | 0.25 | 0.46 | 1.24 |
| Illumination-Day 10 | Property | | / | Clear, colorless liquid | Clear, colorless liquid | Clear, colorless liquid | Clear, light yellow liquid |
| | Impurities (%) | Impurity 1 | / | 0.07 | 0.07 | 0.11 | 0.46 |
| | | Impurity 2 | / | 0.04 | 0.04 | 0.07 | 0.37 |
| | | Maximum single impurity | / | 0.08 | 0.08 | 0.13 | 0.44 |
| | | Total impurities | / | 0.56 | 0.61 | 0.89 | 2.47 |

The results in the above table proved the impact of the pH on the stability of the injectable composition. For Examples 17 to 21, when the pH of the injectable composition was 2.0, the low pH led to a reduced solubility of the compound represented by formula II, and crystals precipitated out of the composition; when the pH of the injectable composition was 5.0, the stability of the composition was reduced, and after the composition was placed under high temperature and illumination conditions, impurities were significantly increased, and the color of the solution changed from colorless to light yellow under illumination; when the pH of the injectable composition was in the range of 3.0 to 4.5 as described in the present disclosure, the composition had good stability, and the property of the composition was not significantly changed.

### Examples 22 to 27

The compound represented by formula II and disodium edetate or calcium disodium edetate were weighed and dissolved in 90% of a prescribed amount of water for injection. The pH of the solution was adjusted with hydrochloric acid or phosphoric acid, and then water for injection was added to the full amount. The solution was filled in a 2 mL brown ampoule, and was flushed with nitrogen before and after filling and sealing. The solution was sterilized at 121°C for 15 minutes to obtain the composition. The results are as shown in Table 5.

**Table 5**

| Ingredient | Example 22 | Example 23 | Example 24 | Example 25 | Example 26 | Example 27 |
|---|---|---|---|---|---|---|
| Compound represented by formula II (g) | 0.2 | 0.5 | 1.0 | 2.0 | 4.0 | 5.0 |
| Disodium edetate (g) | 0.2 | / | / | 0.5 | / | / |
| Calcium disodium edetate (g) | / | 0.2 | 0.4 | / | 0.8 | 1.0 |
| Hydrochloric acid | Appropriate amount | Appropriate amount | / | / | Appropriate amount | / |
| Phosphoric acid | / | / | Appropriate amount | Appropriate amount | / | Appropriate amount |
| pH | 3.5 | 3.5 | 3.5 | 3.5 | 3.5 | 3.5 |
| Add water for injection to make the total volume (mL) | 200 | 200 | 200 | 200 | 200 | 200 |
| Property | Clear, colorless liquid | Clear, colorless liquid | Clear, colorless liquid | Clear, colorless liquid | Clear, colorless liquid | Precipitated crystals observed |

As shown in Table 5, when the concentration of the compound represented by formula II in the injectable composition was 25 mg/mL, crystals precipitated during the preparation of the sample, failing to satisfy the dissolution of the composition, and precipitation or impurities may occur during the preparation and storage process. When the concentration of the compound represented by formula II in the injectable composition was 1 mg/mL or less, a large dose of the injectable solution is required to achieve a sufficient therapeutic effect, leading to an increased difficulty in usage. When the concentration of the compound represented by formula II according to the present disclosure was in the range of 1 mg/mL to 20 mg/mL, the above problems were effectively overcome.

Parts of the components of the present patent have been described in detail above, but it will be apparent to those skilled in the art that such detailed description is set forth only for illustration of exemplary embodiments and should not be construed as limiting the scope of the present patent. Accordingly, the scope of the present disclosure is defined by the following claims and their equivalents.

## Claims

1. An injectable composition, comprising a pharmaceutically acceptable salt of a compound represented by formula I, and a stabilizer, wherein the stabilizer is an edetate,

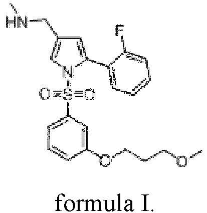

2. The injectable composition according to claim 1, wherein the pharmaceutically acceptable salt is selected from one of fumarate, oxalate, succinate, lactate, mesylate, L-malate, citrate, nitrate, hydrobromide, hydrochloride, phosphate, or sulfate.

3. The injectable composition according to claim 1, wherein the pharmaceutically acceptable salt of the compound of formula I is hydrochloride, represented by formula II below,

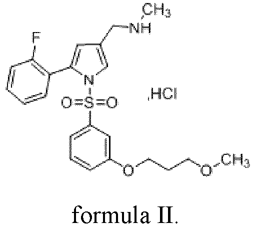

4. The injectable composition according to claim 1, wherein the edetate is one or more of disodium edetate, calcium disodium edetate, tetrasodium edetate, or dicalcium edetate, preferably one or more of disodium edetate or calcium disodium edetate.

5. The injectable composition according to any one of claims 1 to 4, wherein the active ingredient concentration of the pharmaceutically acceptable salt of the compound of formula I is 1 mg/mL to 20 mg/mL.

6. The injectable composition according to any one of claims 1 to 4, wherein the weight ratio of the pharmaceutically acceptable salt of the compound of formula I to the edetate is 1:1 to 20:1, preferably 5:1 to 10:1.

7. The injectable composition according to any one of claims 1 to 4, wherein the injectable composition has a pH of 3.0 to 4.5.

8. The injectable composition according to claim 1, wherein the injectable composition further comprises a pH regulator, and the pH regulator is one or more of hydrochloric acid, sodium hydroxide, citric acid, phosphoric acid, fumaric acid, tartaric acid, or sodium bicarbonate, preferably hydrochloric acid.

9. The injectable composition according to claim 1, wherein the injectable composition comprises the pharmaceutically acceptable salt of the compound represented by formula I, the stabilizer, a pH regulator, and water for injection, wherein the active ingredient concentration of the pharmaceutically acceptable salt of the compound of formula I is 1 mg/mL to 20 mg/mL; the weight ratio of the pharmaceutically acceptable salt of the compound of formula I to the edetate is 1:1 to 20:1, preferably 5:1 to 10:1; and the injectable composition has a pH of 3.0 to 4.5.

10. The injectable composition according to claim 3, wherein the injectable composition contains 2.5 mg of the compound represented by formula II and 0.125 mg to 0.5 mg, preferably 0.25 mg to 0.5 mg, of the stabilizer per 1 mL of the injectable composition.

11. The injectable composition according to any one of claims 1 to 4, wherein the injectable composition is prepared as a solution-type injection.

12. The injectable composition according to claim 11, wherein the solution-type injection is a small-volume injection or a large-volume injection.

13. The injectable composition according to any one of claims 1 to 4, wherein the injectable composition further comprises an osmotic pressure regulator.

14. The injectable composition according to claim 13, wherein the osmotic pressure regulator is one of sodium chloride or glucose.

15. The injectable composition according to claim 13, wherein the osmotic pressure regulator is sodium chloride, and the ratio of the weight of the osmotic pressure regulator to the total volume of the injection solution is 0.6% to 1.0%, preferably 0.6%, 0.7%, 0.8%, or 0.9%.

16. A method for preparing the injectable composition according to any one of claims 1 to 15, wherein the pharmaceutically acceptable salt of the compound of formula I and the edetate are dissolved in a volume of water for injection, and sterilized, to obtain the injectable composition.

17. The method for preparing the injectable composition according to claim 16, wherein the pharmaceutically acceptable salt of the compound of formula I and the edetate are dissolved in the volume of water for injection; the pH of the solution is adjusted to 3.0 to 4.5 with a pH regulator; adding water for injection to make up to volume; and sterilizing the solution by moist heat sterilization for 15 minutes to 30 minutes.

18. The method for preparing the injectable composition according to claim 16 or 17, wherein the pharmaceutically acceptable salt of the compound of formula I and the edetate are added to 80% to 90% of a prescribed amount of water for injection, and stirred until dissolved; the pH of the solution is adjusted to 3.0 to 4.0 with 1 mol/L hydrochloric acid, and water for injection is added to the full amount; and the solution is filtered and flushed with nitrogen, followed by filling, sealing, and moist heat sterilization at 121°C for 15 minutes to 30 minutes.

19. Use of the injectable composition according to any one of claims 1 to 15 in preparation of a medicament for treatment or prevention of peptic ulcer, Zollinger-Ellison syndrome, gastritis, erosive esophagitis, reflux esophagitis, symptomatic gastroesophageal reflux disease, Barrett's esophagitis, functional dyspepsia, Helicobacter pylori infection, gastric cancer, gastric mucosa-assisted lymphoid tissue (MALT) lymphoma, ulcer caused by non-steroidal anti-inflammatory drugs, or hyperchlorhydria or ulcer caused by postoperative stress; or in preparation of a medicament for inhibiting peptic ulcer, acute stress ulcer, hemorrhagic gastritis, or upper gastrointestinal bleeding caused by invasive stress.

20. The use according to claim 19, wherein the peptic ulcer comprises gastric ulcer, duodenal ulcer, or anastomotic ulcer; and the symptomatic gastroesophageal reflux disease comprises non-erosive reflux disease or gastroesophageal reflux disease without esophagitis.
